# EUROPEAN PATENT APPLICATION

(11) **EP 1 839 683 A1**
(43) Date of publication of application: **03.10.2007**
(21) Application number: 06112097.8
(22) Date of filing: 31.03.2006
(51) Int. Cl.: A61L 2/26, A61B 19/00, A61B 19/02, A61C 19/00, A61C 19/02

(54) **Tray for surgical instruments**

(71) Applicant: Stichting Christelijk Algemeen Ziekenhuis Noordwest-Veluwe, 3840 AC Harderwijk (NL)
(72) Inventor: Huenestein, van, Gerrit, 3844 CN, Harderwijk (NL)
(74) Representative: Aalbers, Arnt Reinier

(57) **Abstract**

The invention pertains to a tray (5) for surgical instruments (16), in particular instruments having a lumen, comprising supports (15) for accommodating two or more different instruments (16) and a duct (17) for a washing liquid having an inlet (18) for connecting the duct (17) to a washing liquid supply (9) in a washing machine (1) and one or more outlets (19) for connecting the duct (17) to the lumen(s) of one or more of the instruments (16).

## Description

The invention relates to a tray for surgical instruments, in particular instruments having a lumen, such as laparoscopic instruments. The invention further relates to a washing machine and to a method of disinfecting and sterilising surgical instruments.

International patent application WO 98/50083 relates to divider system (10), which is employed to create small pockets (30, 52) or compartments in a tray (12), case or basket in order to store or support medical instruments (38) during steam sterilisation. As explained in WO 98/50083, it is common practice to put medical instruments in trays or in cases or baskets and place them in high temperature environments for the purpose of sterilisation.

DE 196 11 680 discloses a basket having holders for the surgical tools and their accessories. At least part of the holder (11) has bar shaped mounts (12). The mounts are inserted in openings (13) present in the tools and accessories (8) and seal them off. Only one mount can be provided for each tool or accessory and the mounts are orientated so that openings or ducts in these face downwards.

EP 919 282 relates to an insert (1) comprising a rinsing-water tubular system (2) dockable at the rinsing-water supply system of an automatic washer. The insert comprises a basic module and several separate modules each for a specific kind of utensils to be rinsed, for instance one module for pipettes (Figs. 2 and 3) and two modules for laboratory glassware (Figs. 4-7).

It is an object of the present invention to provide an improved tray for surgical instruments.

To this end, the tray according to the invention comprises supports for accommodating two or more different (kinds of) instruments and a duct for a washing liquid, typically water, having an inlet for connecting the duct to a washing liquid supply in a washing machine and one or more outlets for connecting the duct to the lumen(s) of one or more of the instruments.

It is preferred that at least two of the outlets have mutually different diameters, preferably adapted to the widths or volumes of the lumens of the instruments to which they are to be connected.

It is further preferred that the tray comprises supports for accommodating a (pre-defined) set or subset of surgical instruments for a specific operation, e.g. for treating sinusitis or trauma.

The invention also relates to a washing machine for disinfecting surgical instruments, in particular instruments having a lumen, such as laparoscopic instruments, comprising a frame for supporting, preferably side-by-side, two or more of the trays described above, which frame in turn comprises a duct having two or more water supplies.

Finally, the invention relates to a method of disinfecting and sterilising surgical instruments, comprising the steps of
placing a set or subset of surgical instruments, comprising at least one instrument having a lumen, in a tray comprising a duct for a washing liquid,
connecting the duct to the lumen(s),
placing the tray in a washing machine and connecting the duct to a washing liquid supply,
washing and disinfecting the instruments while in the tray, and preferably
packaging the tray containing the instruments, and sterilising the instruments inside the package.

The tray according to the present invention facilitates thorough cleaning of surgical instruments, in particular of the lumens of hollow instruments, as well as simplified logistics e.g. in and around the sterilisation department of a hospital and the operating theatres, as will be explained in more detail below.

The invention will now be illustrated with reference to the drawings, which schematically show a preferred embodiment of the present apparatus and module.

It is noted that the drawings are not to scale and that details, which are not required for understanding the present invention, may have been omitted. Further, elements that are at least substantially identical or that perform an at least substantially identical function are denoted by the same numeral.

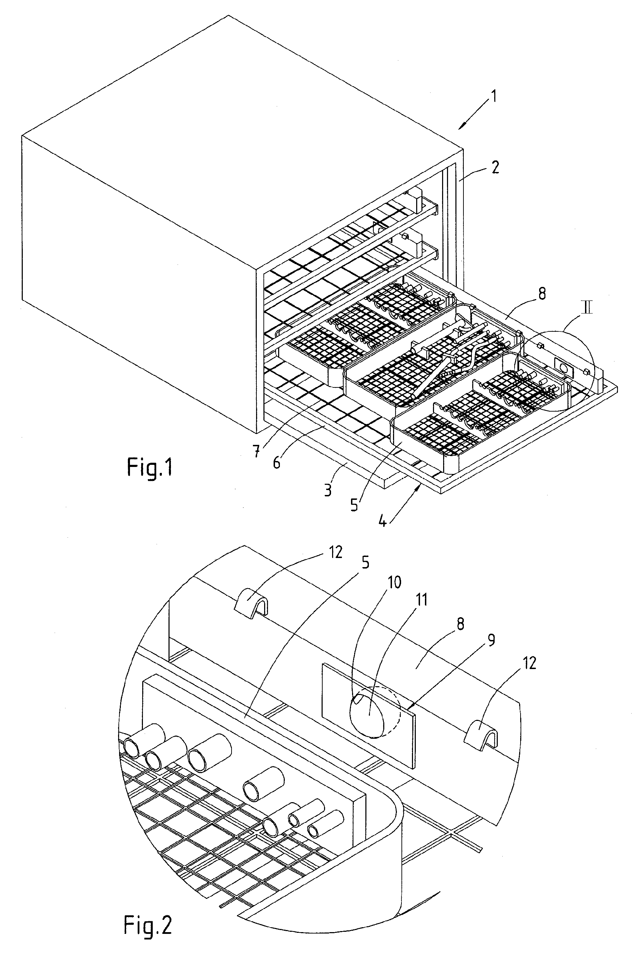

Figure 1 is a perspective view of a washing machine for disinfecting surgical instruments in accordance with the present invention.

Figure 2 is a detail of Figure 1.

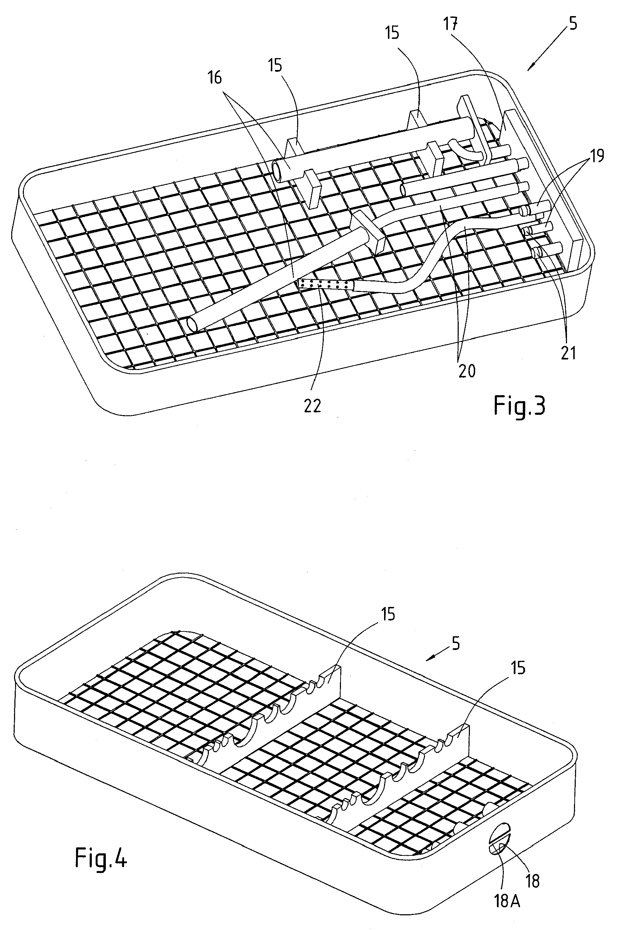

Figures 3 and 4 are perspective views of a tray according to the present invention.

Figure 1 shows a washing machine 1 for disinfecting surgical instruments in accordance with the present invention. The washing machine 1 comprises a casing 2, a front door 3, and one or more frames 4, in this example three frames, slidably received inside the casing 3. Each frame 4 is dimensioned to support a specific number of trays 5 and comprises an outer rim 6, a surface, e.g. a grid 7, for carrying the trays 5, and a distribution duct 8 provided with water supplies 9 corresponding in number to the number of trays 5, i.e. in this case three. Each water supply 9 comprises a circular opening 10, closed by means of a ball 11 urged, from inside the duct 8, e.g. by means of a (helical) spring (not shown), against the circumference of the opening 10. At least when the front door 3 is closed, the ducts 8 are connected to a central water supply (not shown) inside the washing machine 1. Hooks 12 are provided along, e.g. welded to an upper rim of the duct 8, preferably two hooks 12 for each tray 5 positioned on either side of respective openings 10.

As shown in Figures 2 to 4, each of the trays 5, in practice also referred to as e.g. "basket", "sterilisation unit" or STE, comprises supports 15 for accommodating different instruments 16 of a single, pre-defined set or subset of surgical instruments. Such sets (or combinations of subsets) contain all instruments required for a specific operation, e.g. for treating sinusitis or trauma. At least some of the trays 5 comprise a duct, in this example a manifold 17, having an inlet 18, provided with a crossbar 18A and preferably flush with a wall of the tray 5, for connecting the manifold 17 to one of the water supplies 9. The manifold 17 shown in the Figures has a plurality of outlets 19 of various diameters, onto which flexible tubes 20 have been fitted or which have been closed with a stopper 21. In Figure 3, one of the tubes 20 is provided, on its end remote from the manifold 17, with a pipe 22 having a plurality of small openings evenly distributed over its surface. In this example, the frames 4 and the trays 5 are made of stainless steel. The flexible tubes 20 and the stoppers 21 are made of a material, such as silicone, resistant to temperatures and pressures of at least 130°C and 300 kPa, respectively.

In modern practice, a surgeon, at the onset of an operation, is provided with one or more trays containing the set of instruments required for that specific operation. Such a set comprises, for e.g. placing a dynamic hip screw, one basis tray containing instruments for making incisions and closing the wound and one special tray containing straightening tools, drills, and cutters, some of which are hollow. For the treatment of sinusitis, a single tray containing nose specula, tweezers, suction tubes, and a trocar, typically suffices.

During surgery, the instruments are taken from the trays and, after use, put back in the respective tray(s). After completion of the operation, the trays are transported to disinfection and sterilisation facilities. There, the instruments are disassembled (if applicable) and pre-washed and, subsequently, the tubes are connected to corresponding instruments, i.e. smaller diameter tubes to instruments having a smaller lumen and larger diameter tubes to instruments having a larger lumen. Where appropriate, a pipe (22) is inserted into a corresponding lumen. In some embodiments, instruments are urged onto an outlet opening of the manifold, as is shown in Figure 3, which opening, in such cases, is preferably be provided with a short flexible tube, a gasket or the like.

Subsequently, the trays are placed on the frames of the washing machine by hooking the rim of each of the trays under the hooks, with the manifold facing the corresponding opening 10, and lowering the tray. As a result, the crossbar in the inlet opening of the manifold will urge the respective ball inwards thus establishing fluid communication between de duct and the manifold and hence between the duct and the lumens of the instruments.

When all frames are filled with trays, the washing machine is closed and the washing and disinfection cycle is initiated. Washing and disinfecting typically includes rinsing with cold water, adding a detergent, rinsing with successively hot water at e.g. 60°C, cold water, and hot water at e.g. 90°C, and finally drying the instruments with hot air at e.g. 120°C. During this process, the lumens are constantly flushed with water respectively air.

Next, the contents of the trays are checked, each of the trays is wrapped in sterilisation paper, and the obtained package is sterilised, e.g. at approximately 120°C and 200 kPa (if fragile instruments are contained in the tray) or at 130°C and 300 kPa. Finally, the sterilised packages are stored or returned to surgery.

In this preferred example, the instruments including the lumens are thoroughly cleansed and disinfected. Furthermore, logistics are simplified in that a washing and disinfection cycle can be started as soon as sufficient trays have arrived to fill a machine, as opposed to having to wait until several inserts or modules dedicated to a single, specific kind of equipment instruments, such as tweezers or trocars, have been filled, and the risk of incorrect composition of the (sub)sets is reduced considerably. Put differently, the instruments belonging to one specific set remain together at all stages of surgery, disinfection, and sterilisation (which chain may repeat itself upto six times in one day), thus simplifying logistics and reducing errors.

The invention is not restricted to the above-described embodiments, which can be varied in a number of ways within the scope of the claims. For instance, one may choose a standard manifold for various (sub)sets of instruments (preferably sealing outlets that are not used) or two or more manifolds tailored to specific (sub)sets of instruments. Further, it is preferred that the length, width and height of the trays are in a range of, respectively, 40 to 60 cm, 20 to 35 cm, and 4 to 15 cm, more preferably that these dimensions are in accordance with ISO, e.g. 48x30x6 cm, and/or DIN (in particular no. 58 952), e.g. 48x25x5 cm, standards.

## Claims

1. Tray (5) for surgical instruments (16), in particular instruments having a lumen, comprising supports (15) for accommodating two or more different instruments (16) and a duct (17) for a washing liquid having an inlet (18) for connecting the duct (17) to a washing liquid supply (9) in a washing machine (1) and one or more outlets (19) for connecting the duct (17) to the lumen(s) of one or more of the instruments (16).

2. Tray (5) according to claim 1, wherein the duct (17) comprises a plurality of outlets (19) for connecting the duct (17) to the lumens of a plurality of instruments (16).

3. Tray (5) according to claim 1 or 2, wherein at least one of the outlets (19) of the duct (19) is provided with a flexible tube (20).

4. Tray (5) according to any one of the preceding claims, wherein at least two of the outlets (19) and corresponding tubes (20) have different diameters.

5. Tray (5) according to claim 3 or 4, wherein at least one of the tubes (20) is provided, on its end remote from the duct (17), with a rigid element (22) provided with a plurality of openings.

6. Tray (5) according to any one of the preceding claims, comprising supports (15) for accommodating a set or subset of surgical instruments (16) required for a specific operation.

7. Washing machine (1) for disinfecting surgical instruments (16), in particular instruments having a lumen, comprising a frame (4) for supporting two or more trays (5) according to any one of the preceding claims, which frame (4) in turn comprises a duct (8) having two or more one water supplies (9).

8. Washing machine (1) according to claim 7, wherein the duct(s) (8) extend(s) at least substantially horizontally.

9. Method of disinfecting and sterilising surgical instruments (16), comprising the steps of
placing a set or subset of surgical instruments (16), comprising at least one instrument having a lumen, in a tray (5) comprising a duct (17) for a washing liquid,
connecting the duct (17) to the lumen(s),
placing the tray (5) in a washing machine (1) and connecting the duct (17) to a washing liquid supply, and
washing and disinfecting the instruments (16) while in the tray (5).

10. Method according to claim 9, comprising, after disinfecting, the steps of
packaging the tray (5) containing the instruments (16), and
sterilising the instruments (16) inside the package.
